(19) Europäisches Patentamt / European Patent Office / Office européen des brevets

(11) **EP 4 332 562 B1**

(12) **EUROPEAN PATENT SPECIFICATION**

(45) Date of publication and mention
of the grant of the patent:
**08.07.2026 Bulletin 2026/28**

(21) Application number: **22461602.9**

(22) Date of filing: **02.09.2022**

(51) International Patent Classification (IPC):
**G01N 27/414** (2006.01)

(52) Cooperative Patent Classification (CPC):
**G01N 27/4145**

(54) **A MESFET BIOSENSOR AND A BIOSENSING KIT**

MESFET BIOSENSOR UND BIOSENSOR-KIT

BIOCAPTEUR MESFET ET KIT DE BIODÉTECTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**06.03.2024 Bulletin 2024/10**

(73) Proprietor: **IQ Biozoom Sp. z o.o.**
**37-450 Stalowa Wola (PL)**

(72) Inventors:
• **KACZMARSKI, Jakub**
**Warszawa (PL)**
• **DARDZINSKA, Dorota**
**Warszawa (PL)**
• **KACZMARSKA, Katarzyna**
**Warszawa (PL)**

(74) Representative: **Kancelaria Eupatent.pl Sp. z o.o.**
**ul. Jana Kilinskiego 193 lok. 2.13**
**93-106 Lodz (PL)**

(56) References cited:
**US-A1- 2010 194 409    US-A1- 2015 276 667**
**US-A1- 2016 202 208**

• **JESEUNG OH ET AL: "A carbon nanotube metal semiconductor field effect transistor-based biosensor for detection of amyloid-beta in human serum", BIOSENSORS AND BIOELECTRONICS, vol. 50, 1 December 2013 (2013-12-01), Amsterdam , NL, pages 345 - 350, XP055366998, ISSN: 0956-5663, DOI: 10.1016/ j.bios.2013.07.004**
• **KACZMARSKI JAKUB ET AL: "IGZO MESFET with enzyme-modified Schottky gate electrode for glucose sensing", JAPANESE JOURNAL OF APPLIED PHYSICS, vol. 58, no. 9, 13 May 2019 (2019-05-13), JP, pages 090603, XP093021574, ISSN: 0021-4922, Retrieved from the Internet <URL:http://stacks.iop.org/1347-4065/58/i=9/ a=090603?key=crossref. fd81b2ec9e54f9c20cfa4b163fd28e3f> DOI: 10.7567/1347-4065/ab1a65**
• **ZHAO CHUMIN ET AL: "Two-Dimensional Numerical Simulation of Bottom-Gate and Dual-Gate Amorphous In-Ga-Zn-O MESFETs", IEEE ELECTRON DEVICE LETTERS, vol. 35, no. 1, 1 January 2014 (2014-01-01), USA, pages 75 - 77, XP093311778, ISSN: 0741-3106, DOI: 10.1109/ LED.2013.2289861**

**Description**

TECHNICAL FIELD

The present invention relates to a biosensor comprising a metal-semiconductor field-effect transistor, and to a biosensing kit suitable for detection of various target substances present in a liquid sample, including biological substances such as proteins, sugars, antigens, nucleic acids sequences etc.

BACKGROUND

Field-effect transistors (FETs), including metal-semiconductor field-effect transistors (MESFETs), are used as biosensor components to detect a target substance in liquid samples - including samples of biological liquids such as saliva, urine, sweat, or blood. Typically, methods for detection based on such biosensors involve exposing a sensitive area of the transistor to the liquid sample; said sensitive area typically comprises functionalization species that can interact with the target substance contained within the liquid sample. During the detection procedure, said interaction affects the source-drain current in the transistor channel, and based on that presence and/or concentration of the target substance in the sample can be determined.

Among the known designs of the biosensors based on the FET-type transistors, problems associated with the influence of the external factors during the assay occurs. Namely, the temperature or pH of samples and other so-called external interfering factors may distort the measurement results, causing unreliability of the obtained data.

Various attempts have been taken to address the above inconveniences. *Inter alia,* US patent application US2011/0132773A1 describes an apparatus for detecting biological substances in a fluid flow, comprising two transistors (FET), one transistor acting as a reference element, and the other transistor acting as a measuring sensor-with a gate electrode sensitive to a target substance. The source electrode of one transistor and the drain electrode of the other transistor are connected to one another and to a signal line. In operation, a voltage is applied between the drain electrode of the first transistor and the source electrode of the second transistor, and a current of the signal line is the difference in the channel currents of the two transistors (differential current). In this configuration, compensation takes place directly in the sensor (by wiring the measuring transistor, to the reference transistor). This way influence of certain interfering factors can be reduced, improving the signal-to-noise ratio.

A publication "A carbon nanotube metal semiconductor field effect transistor-based biosensor for detection of amyloid-beta in human serum" (by Oh Jeseung et al in Biosensors and Bioelectronics 50 (2013): 345-350) discloses a carbon nanotube (CNT) MESFET transistor for use as a biosensor, formed on a semiconductive substrate that functions as a bottom gate electrode and extends across the whole width of the transistor, including the whole width of the channel, which is made of carbon nanotubes.

A publication "IGZO MESFET with enzyme-modified Schottky gate electrode for glucose sensing" (by Jakub Kaczmarski et al in Japanese Journal of Applied Physics 58.9 (2019): 090603) describes the development of a glucose sensor through the immobilization of an enzyme (glucose oxidase) into the gate of an In-Ga-Zn-O thin film transistor in a MESFET configuration with Ru-Si-O acting as a Schottky gate electrode.

US2010194409A1 relates to a biosensor where the capture molecules are immobilised on a sensing unit of an extended gate field effect transistor, which includes a semiconductor on a conductive substrate surrounded by an insulator. The semiconductor is connected via a conductive wire to the gate of a MOSFET. A MESFET is suggested as alternative transistor.

US2016202208A1 relates to a multiplexing detection system of a dual gate ion-sensitive MISFET bio sensor.

US2015276667A1 relates to a MISFET-based sensor array.

SUMMARY OF THE INVENTION

The present invention aims to provide an alternative apparatus for detecting substances, that is capable of detecting a target substance, wherein at least some of the interfering factors can be compensated, thereby improving a signal-to-noise ratio for the obtained results.

The object of the invention is a biosensor according to the appended claims.

In one aspect, the invention relates to a biosensor comprising a detection transistor of a metal-semiconductor field-effect (MESFET) type, for detecting of a target substance. The detection transistor comprises: a source electrode, a drain electrode, a first gate electrode connected with a sensitive substrate comprising functionalization species for bonding the target substance, such that the first gate electrode connected with said substrate is sensitive to the target substance, and a channel for transmitting a drain-source current between the source electrode and the drain electrode. The detection transistor further comprises a second gate electrode physically separated from the first gate electrode by the channel.

Such arranged second gate electrode allows for modulation of the drain-source current which in turn enables adequate compensation for the influence of external factors including presence of electrolytes or nitrogenous species in the sample (which could interfere with the obtained results). The biosensor comprising said detection transistor exhibits improved stabilization of its operation point, resulting in increased reliability of the assay outcome. This is achieved by the control (with the use of microcontroller) of the transconductance of the channel layer of TFT. As a result the changes of the IDS of biosensing transistors corresponds to the changes of monitored species concentration in the analyte.

The second gate electrode is arranged to modulate the drain-cource current in the channel. This provides further stabilization of the operating point of the detection transistor.

The detection transistor further comprises an isolating substrate on which the channel is arranged. The substrate provides protection of the transistor form the external conditions.

The second gate electrode is arranged between the isolating substrate and the channel. Such arrangement enables to protect the second gate electrode form the external conditions whilst enabling the second gate electrode to modulate the the drain-cource current in the channel.

The biosensor may further comprise an encapsulation arranged such that it exposes the first gate electrode. The encapsulation provides further protection of the transistor from environmental negative affections such as moisture or oxygen while the connection of the first gate electrode with the sensitive substrate can be accomplished.

The first gate electrode is conneced with the sensitive substrate via a conductive path, preferably selected from the group consisting of conductive film, wiring, junction connector, and electrolytic connection. The sensitive substrate configured to be detachably attached to the first gate electrode is arranged as disposable. Thus, the transistor itself can be used multiple times, and the disposable sensitive substrate once used, can be replaced with the new one - for a new assay. It is an especially convenient solution when it comes to experienced users or the ones who use the device on the regular basis, e.g. on daily basis for glucose monitoring.

The sensitive substrate is arranged on a disposable strip comprising a sheet of material with the functionalization species, for detachably connecting with the first gate electrode. The sensitive substrate disposed on a disposable strip is less prone to damage, easy to chandler, and easier combinable with the first gate electrode of the detection transistor.

The biosensor may further comprise a reference transistor of a metal-semiconductor field-effect (MESFET) type, the reference transistor comprising: a source electrode, a drain electrode, a first gate electrode connected with a insensitive substrate without the functionalization species, such that the first electrode connected with said substrate is insensitive to the target substance, a channel for transmitting a drain-source current between the source electrode and the drain electrode, and a second gate electrode physically separated from the first gate electrode by the channel. The detection transistor and the reference transistor are electrically connected to one another by the second gate electrodes. The reference transistor provides further stabilization of the operating point of the biosensor. The reference transistor can be configured to measure the various external factors such as pH of the sample thereby providing more accurate assay results. Further, the arrangement of the second gate electrodes of the detection and reference sensors as described above provides increase in the measurement stability, by selecting the operation point and thus increase in the measurement accuracy, sensitivity and specificity of the device.

The second gate electrode of the detection transistor and the second gate electrode of the reference transistor can be arranged as a common second gate electrode of both transistors, formed from a coherent piece of material. Such design provides simplified production for the biosensor device, as both secont gate eelctrodes can be produced it one step, leading to the cost and failure rate reduction.

The detection transistor and the reference transistor can be arranged on a common substrate and covered with a common encapsulation. Such design further simplifies the manufacture of the biosensor, as providing the substrate and encapsulating can be accomplished faster.

The common second gate electrode may extend from the channel of the detection transistor to the channel of the reference transistor, wherein said channels separate the common second gate electrode from the first gate electrodes of both transistors. Such design provides adequate modulation of the drain-source current of both, the detection transistor and the reference transistor.

In another aspect, the present invention relates to a biosensing device comprising the biosensor as described herein and further comprising: a control unit for processing measured data; a power supply unit for powering the device; and a data display unit for visualizing a detection result.

In yet another aspect, the present invention relates to a biosensing kit comprising: the biosensor as described herein, and a disposable substrate unit comprising: the sensitive substrate with functionalization species sensitive to the target substance, detachably connectable with the first gate electrode of the detection transistor.

In a further aspect, the present invention relates to a biosensing kit comprising the biosensor as described herein and a disposable substrate unit comprising the sensitive substrate with functionalization species sensitive to the target substance, detachably connectable with the first gate electrode of the detection transistor, and the insensitive substrate without functionalization species sensitive to the target substance, detachably connectable with the first electrode gate of the reference transistor. The kit allows for using the biosensing unit multiple times - the utilized strip can be replaced with a new one, each time the new assay is to be carried out.

Further aspects and features of the present invention are described in following description of the drawings.

BRIEF DESCRIPTION OF DRAWINGS

Aspects and features of the present invention will become apparent by describing, in detail, exemplary embodiments of the present invention with reference to the attached drawings, in which:

Fig. 1A shows an exemplary (not in accordance with the present invention) detection transistor in a cross-sectional view;
Fig. 1B shows an embodiment of the detection transistor in a cross-sectional view;
Figs 2A - 2C show a general principle of operation of the detection transistor;
Fig. 3 shows a biosensing unit comprising the detection transistor and a reference transistor, not according to the present invention;
Fig. 4 shows a biosensor comprising the biosensor unit according to the present invention;
Fig. 5 shows a diagram representing how to use a biosensing kit;
Fig. 6A schematically shows (in enlargement) the biosensing kit with a disposable substrate connected with the biosensing unit;
Fig. 6B shows examples of energy band diagrams of a first gate electrode and channel of the detection transistor in pinch-off (0% glucose) and conducting (>0% glucose);
Fig. 7 shows an electrical schematic of the biosensor unit comprising the detection transistor and the reference transistor according to the present invention;
Fig. 8 shows output characteristics of the detection transistor.

DETAILED DESCRIPTION OF EMBODIMENTS

Reference will now be made to embodiments, examples of which are illustrated in the accompanying drawings. Aspects and features of the embodiments will be described with reference to the accompanying drawings. In the drawings, like reference numerals denote like elements. The present invention, however, may be embodied in various different forms and should not be construed as being limited only to the illustrated embodiments. Rather, these embodiments are provided as examples so that this disclosure will be thorough and complete, and will fully convey the aspects and features of the present invention to those skilled in the art. It shall be understood that not all of the features shown in the embodiments are essential and the scope of the protection is defined not by means of literally shown embodiments, but by the features provided in the claims.

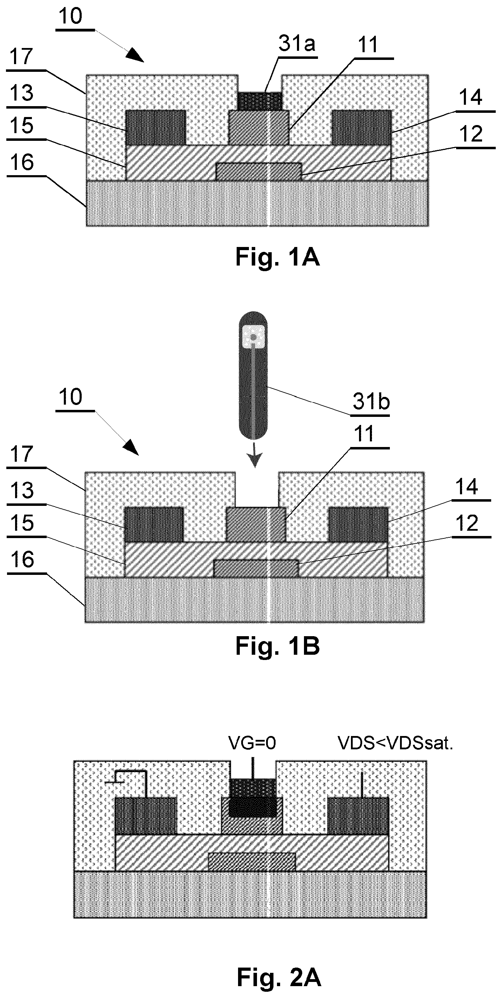

Figs 1A, 1B show respectively a first (not in accordance with the present invention) and a second (in accordance with the present invention) embodiment of a detection transistor 10 suitable for use in a biosensor according to the invention. The detection transistor 10 is a metal-semiconductor field-effect transistor (MESFET) suitable to be used as a component of the biosensor device for detecting one or more target substances during a single assay. The biosensor may be configured to detect target substances such as sugar (e.g. glucose), proteins, or amino acids sequences (e.g. D/L-tryptophan (D/L-Trp), D/L-tyrosine (D/L-Tyr) and D/L-phenylalanine (L-Phe) ), nucleotide sequences, etc. The biosensor is configured to detect whether the target substance is contained in a sample e.g. a sample of saliva, blood, urine, sweat, or another biological fluid taken for instance from a human or animal body.

The detection transistor 10 comprises a source electrode 13 and a drain electrode 14 connected by a semiconducting channel 15 such that, during the assay, a drain-source current ($I_{DS}$) can flow through the channel 15, between the source and drain electrodes 13, 14. Further, the detection transistor 10 comprises a first gate electrode 11 placed over the channel 15. The first gate electrode 11 is configured as being sensitive to the target substance for the time of assay. Furthermore, the detection transistor 10 comprises a second gate electrode 12 configured to modulate the drain-source current of the channel 15 during the assay, to provide compensation, at least to some extent, of the influence of interfering factors such as electrolytes, nitrogenous, etc. for the detection results. Namely, the second gate electrode 12 provides stabilization of the operating point of the transistor, by keeping the transconductance of the channel constant. The second gate electrode 12 is separated from the first gate electrode 11 by the channel 15 and positioned at an opposite side of the channel 15 with respect to the first gate electrode 11. To achieve proper operation, both gate electrodes 11 and 12 are Schottky junctions to channel layer.

The detection transistor 10 is arranged on an insulating substrate 16, therefore the second gate electrode 12 and the channel 15 are in contact with the isolating substrate 16 and physically separated from the remaining elements (11, 13, 14) of the detection transistor 10.

The source electrode 13, the drain electrode 14, and the first gate electrode 11 are arranged on the channel 15, wherein the first gate electrode 11 is arranged between the source electrode 13 and the drain electrode 14.

The source and drain electrodes 13, 14 may be made of various metals which can serve as ohmic contacts, such as molybdenum (Mo) or aluminum (Al). The first and second gate electrodes 11, 12 are Schottky contacts to the channel 15, and therefore are preferably be made of various high work-function materials such as $AgO_x$ or $PtO_x$, $PdO_x$. The channel 15 is made of an oxide semiconductor such as In-Ga-Zn-O (IGZO) that exhibits electron mobility above 10 $cm^2$ $V^{-1}$ $s^{-1}$, amorphous microstructure and easiness of large area uniform thin film formation even at room temperature, and provides stable, low operation voltages and high switching speeds of the detection transistor 10 - all of such the advantages are desired in the biosensor applications.

The detection transistor 10 is preferably covered with an encapsulation 17, e.g. made of a polymer, such as SU-8 (i.e. an epoxy-based negative photoresist), PDMS (poly(dimethylsiloxane)), or PMMA (poly(methyl methacrylate)) to preserve the transistor components from environmental conditions that could negatively affect the materials of the transistor 10. The encapsulation 17 can prevent, or at least restrict the penetration of moisture and oxygen into the transistor. The encapsulation 17 is arranged so as to expose the area of the first gate electrode 11 that is configured to become sensitive to the target substance, thus, allowing the target substance to be captured by said area of the first gate electrode 11 - if provided as sensitive.

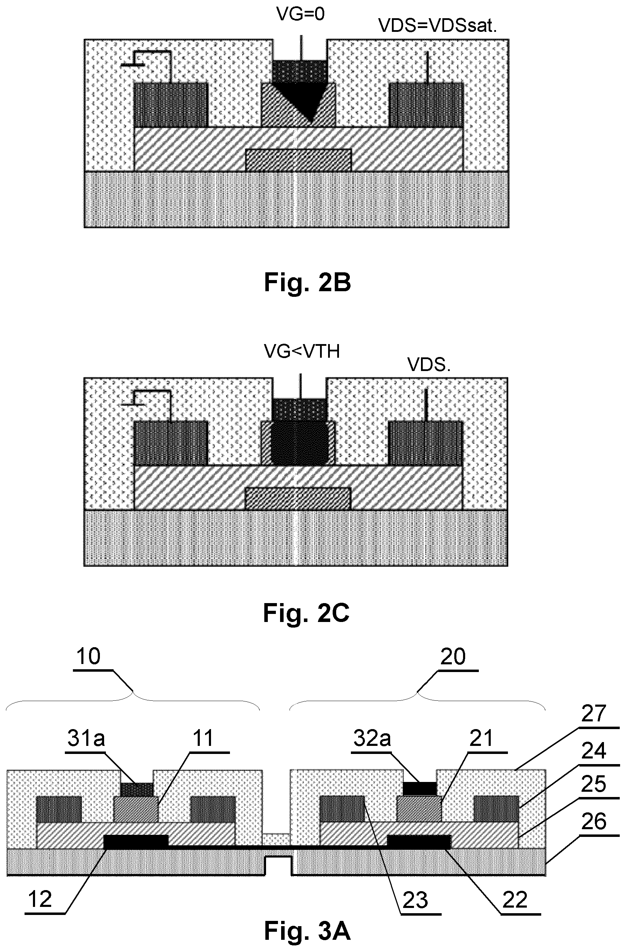

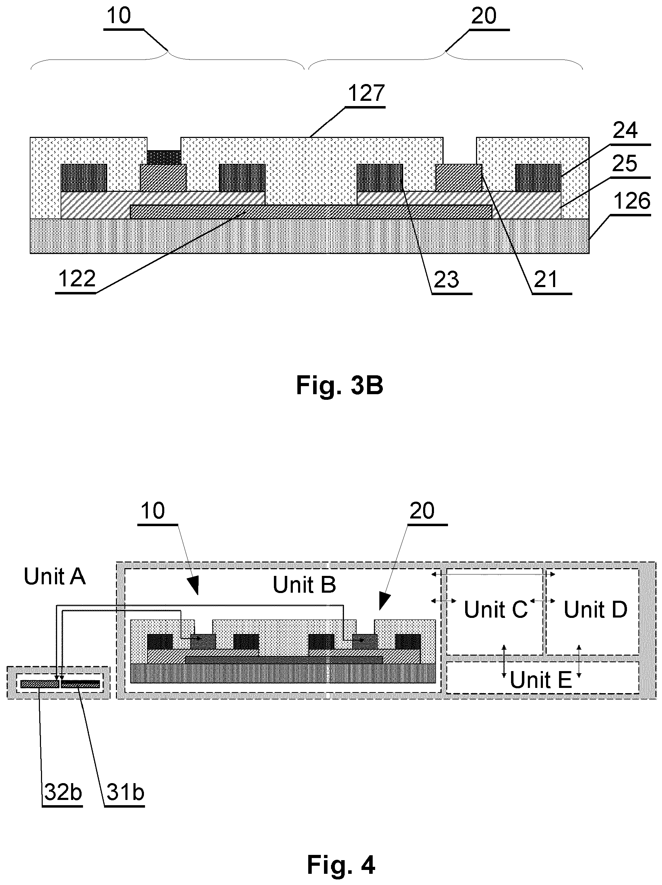

The present invention further pertains to a biosensor device, such as shown in an embodiment of Fig. 4. The biosensor comprises a biosensing unit B, wherein the biosensing unit B may essentially consist of the detection transistor 10. Alternatively, the biosensing unit B may comprise the detection transistor 10 and a reference transistor 20 connected with each other, for example as shown schematically in Figs. 3A and 3B.

In one specific embodiment, the reference transistor 20 of the biosensing unit B may have the same design as the detection transistor 10. The reference transistor 20 comprises a source electrode 23 and a drain electrode 24 connected by a channel 25 such that, during the assay, a drain-source current ($I_{DS}$) can flow through the channel 25, between the source and drain electrodes 23, 24. Further, the reference transistor 20 comprises a first gate electrode 21 placed over the channel 25. The first gate electrode 21 is configured to be insensitive to the target substance for the time of the assay. Also, the reference transistor 20 comprises a second gate electrode 22 configured to modulate the drain-source current of the channel 25, and thus stabilizing the operating point of the reference transisitor. The second gate electrode 22 is physically separated from the first gate electrode 21 by a material of the channel 25. Preferably, the reference transistor is arranged on an insulating substrate 26, which can be made of $SiO_2$, or another insulating material such as plastic foil, e.g. PET foil. The source electrode 23, the drain electrode 24, and the first gate electrode 21 are arranged on the channel 25, wherein the first gate electrode 21 is arranged between the source electrode 23 and the drain electrode 24. The source and drain electrodes 23, 24 may be made of various metals which can serve as ohmic contacts; for instance, molybdenum (Mo) or aluminum (Al). The first and second gate electrodes 21, 22 are Schottky contacts to the channel 25, and they may be made of various materials such as $AgO_x$ or $PtO_x$, $PdO_x$ etc. The channel 25 is made of an oxide semiconductor such as In-Ga-Zn-O (IGZO), as this semiconductor exhibits electron mobility above 10 $cm^2$ $V^{-1}$ $s^{-1}$, amorphous microstructure and easiness of large area uniform thin film formation even at room temperature, and providing stable, low operation voltages and high switching speeds of the reference transistor 20 - all of such the advantages are desired in the biosensor applications.

Furthermore, the detection transistor 10 and the reference transistor 20 arranged together in the biosensing unit B may have the same proportions, the same geometry, the same architecture, the same dimensions, and they can be made of the same materials, except for the first gate electrode 21 of the reference transistor 20 which is configured to be insensitive to the target substance, especially for the time of assay.

The biosensing unit B of the biosensor, comprising the detection transistor 10 and the reference transistor 20 is shown in Fig. 4, and Figs. 3A and 3B in isolation from the remaining units of the device. The device can further comprise a control unit C, a power supply unit D and optionally a display unit E, to register and process signals from the biosensing unit B, and to visualize the obtained results of detection. The respective units C, D, E may be made of a known hardware, such as a microcontroller powered from a power source, as well as a display panel for data visualization, operable by suitable software for data processing.

As show in Fig. 3A, within the biosensing unit B of the biosensor, the detection transistor 10 and the reference transistor 20 have their second gate electrodes 12, 22 electrically connected, so that the second gate electrode 12 of the detection transistor 10 and the second gate electrode 22 of the reference transistor 20 can be maintained under the same bias, and preferably under the same constant bias during the whole assay procedure. In one preferred embodiment of the biosensing unit B, shown in Fig. 3B, the second gate electrode of the detection transistor 10 and the second gate electrode of the reference transistor 20 are arranged as a common second gate electrode 122 - formed by a coherent piece of material. The common second gate electrode 122 extends between the channels 15, 25 of both transistors 10, 20 so that the common second gate electrode 122 is physically separated by those channels 15, 25 from the first gate electrode 11 of the detection transistor 10 and the first gate electrode 21 of the reference transistor 20, respectively. The transistors 10 and 20 may be arranged on the common substrate 126 and covered with a common encapsulation 127 exposing the first gate electrodes 11, 21 of both transitosrs, the detection transistor 10, and the reference transistor 20. Further, the biosensing unit B may comprise a multiple of the pair: detection transistor-reference transistor; this provides further increase in sensitivity of the assay.

The first gate electrode 11 of the detection transistor 10 is connectable with a sensitive substrate 31a, 31b with functionalization species - suitable for interaction with the target substance. The functionalization species of the sensitive substrate 31a, 31b may be chemical molecules e.g. enzymes, antigens, nucleotide sequences e.g. DNA, or RNA, amino-acids sequences, or chemical groups, e.g. -COOH, -NH$_2$, -OH, which are capable of interaction with the target substance. For example, for the target substance being glucose, the sensitive substrate 31a, 31b may comprise glucose oxidase molecules serving as the functionalization species. The sensitive substrate 31a, 31b may also comprise a base material (inert to the target substance) - for suitable adherence of the functionalization species within the sensitive substrate 31a, 31b.

The first gate electrode 21 of the reference transistor 20 is connectable with an insensitive substrate 32a, 32b (Figs 3A, 4) without functionalization species, thus, the insensitive substrate 32a, 32b is unsuitable for interaction with the target substance. However, the insensitive substrate 32a, 32b is configured to detect at least one of external factors which may have an impact to the detection - distorting the results. For example, the insensitive substrate 32a, 32b may be configured to measure pH of the sample, thereby providing reference that enables to minimize the influence of interferents on the measurement results Preferably, the insensitive substrate 32a, 32b is made of the same base material connected to the electrode 21 as the base material of the sensitive substrate 31a, 31b -connected to the electrode 11 (however, the base material of the insensitive substrate 32a, 32b does not comprise the functionalization species).

The base material of both sensitive and insensitive substrate 31a, 31b, 32a, 32b may be composed for example of polymer, resin, or oligomer; e.g. the base material may be made of conductive membrane polymer, e.g. Nafion®, directly attached (not in accordance with the present invention) to the first gate electrode 11, 21; for the sensitive substrate 31a, 31b, the membrane polymer may comprise enzymes (e.g. glucose oxidase for interaction with glucose).

Connection of the first gate electrode 11 of the detection transistor 10 with the sensitive substrate 31a, as well as connection of the first gate electrode 21 of the reference transistor 20 with the insensitive substrate 32a, 32b can be accomplished either by physical or chemical binding so that the substrate 31a, 32a - once disposed on the first gate electrode 11, 21 - is undetachable (not in accordance with the present invention), and thereby the biosensing unit B of the biosensor is disposable. This is shown schematically in Fig. 1A for the detection transistor 10, and in Fig. 3A for the biosensing unit B - where the sensitive substrate 31a, and insensitive substrate 32a are disposed as layers directly on the first gate electrode 11, 21 respectively, wherein the sensitive substrate 31a, as well as insensitive substrate 32a, may be bonded to the first gate electrode 11, 21 via, e.g., chemical bonding, such as covalent bonds, ionic bonds, etc.

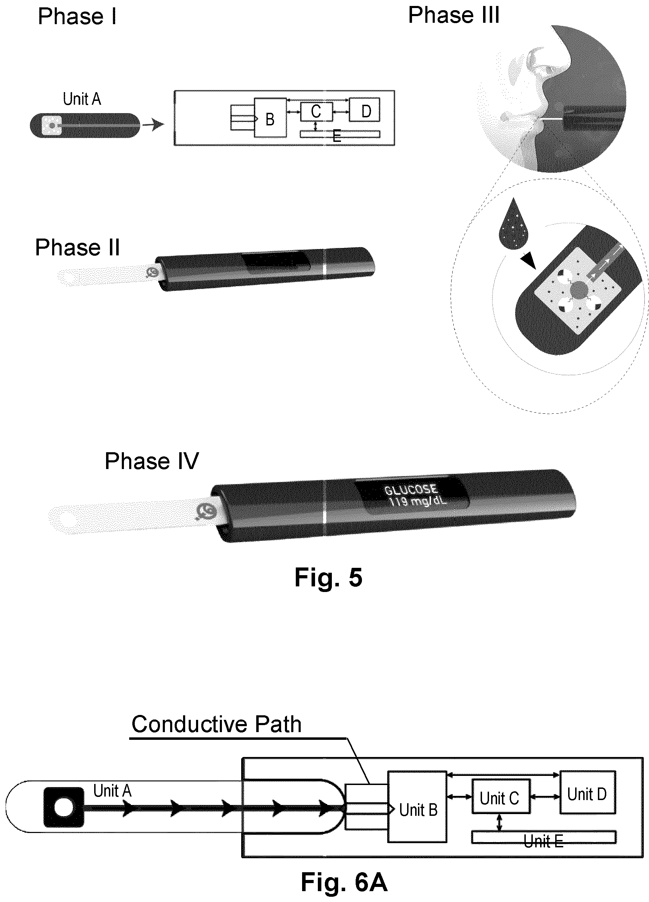

According to the present invention, the substrates 31b, 32b can be detachably connected to the respective first gate electrodes 11, 21 - via a conductive path (Fig. 6A) which allows for repeatedly connecting and disconnecting of the first gate electrode 11 and the respective substrate (sensitive and insensitive 31b, 32b) making the sensitive and insensitive substrate 31b, 32b disposable. The advantage of disposable substrates 31b, 32b, is that the biosensing unit B of the biosensor, and thus, all the transistors can be used multiple times - to conduct multiple assays during the lifetime of the biosensing device. This can considerably reduce the cost of single assays and limit waste generation.

The conductive path - making both sensitive and insensitive substrates 31b, 32b disposable, may take various forms, including but not limited to conductive films, wiring, junction connector, an electrolytic connection, etc. Further, the detachable disposable substrates 31b, 32b may have together a form of a single disposable strip (Fig. 5 - Unit A) comprising a sheet of material for example made of the base material, or comprising the base material with arranged thereon a sensitive zone with the functionalization species, and an insensitive zone without functionalization species, such that both sensitive substrate 31b and insensitive substrate 32b can be simultaneously subjected to interaction with a single sample suspected to comprise the target substance. This provides unified conditions of the assay for the whole biosensing unit B of the biosensor, as shown schematically in Fig. 4.

The assay for detection of the target substance with a biosensing kit comprising the biosensor with the biosensing unit B, and the disposable unit A comprising the sensitive substrate 31b and optionally further comprising insensitive substrate 32b (within the diposable unit A) - is shown schematically in Fig. 5. In phase one (I), the strip (unit A) with the sensitive substrate 31b and the biosensor are provided (for the example where the biosensing unit comprises only the detection transistor 10 - the detachable strip comprises only the sensitive substrate 31b, and for the example where the biosensing unit further comprises the reference transistor 20 - the detachable strip further comprises the insensitive substrate 32b, which are provided in two zones of the strip). In phase two (II), the user attaches the disposable strip with the biosensing unit using the conductive path such that the disposable strip is respectively connected with the first gate electrode 11 of the detection transistor 10, and optionally with the first gate electrode 21 of the reference transistor 20 - if present, as schematically shown in Fig. 6A. In phase three (III), the user brings the sample (suspected to comprise the target substance) in contact with the detachable strip - both zones, the sensitive and insensitive at once. For example, a sample of saliva can be drawn directly from the mouth by holding the strip in the user's mouth for a specified period of time, typically from 1 to several seconds (as shown in Fig. 5 - phase III). The units C-E of the biosensor conduct signal gathering and calculation, wherein the result can be directly displayed on a screen of the biosensor as shown in Fig. 5 - phase four (IV). Furthermore, the obtained data can be wirelessly transferred to external device (such as mobile phone, tablet, etc. - not shown) and processed externally by dedicated applications. The corresponding assays can be conducted for the samples in various forms e.g. blood, sweat, urine - wherein the sample can be brought into contact with the sensitive substrate 31b (and optionally with the insensitive substrate 32b, if provided in the disposable strip - unit A) - prior, during, or after connecting the unit A with the unit B of the biosensing kit - by using the conductive path.

**Detection method:**

The assay is based on measuring the output characteristics: $I_{DS}$ (drain-source current) = f($V_{DS}$) (drain-source voltage) of the detection transistor 10. The drain current ($I_{DS}$) changes as a function of the concentration of the target substance, which is attributed to the changes of the electrical potential (modulation of the depletion region at the gate-channel Schottky region) on the first gate electrode 11, 31a, 31b and corresponds with the electrochemistry of interactions of the functionalization species of the sensitive substrate 31a, 31b and the target substance.

For instance, for glucose detection, the enzyme (glucose oxidase) can be contained in the sensitive substrate for selectively oxidizing glucose. Due to the oxidation reaction, gluconolactone and $H_2O_2$ particles are created as the reaction products, as described in Equation 1:

$$Glucose + O_2 \xrightarrow{(glucose\ oxidase)} gluconolactone + H_2O_2 \qquad (1)$$

Next, the $H_2O_2$ dissociates into protons ($H^+$), electrons ($e^-$), and $O_2$, as described in Equation 2:

$$H_2O_2 \rightarrow O_2 + 2H^+ + 2e^- \qquad (2)$$

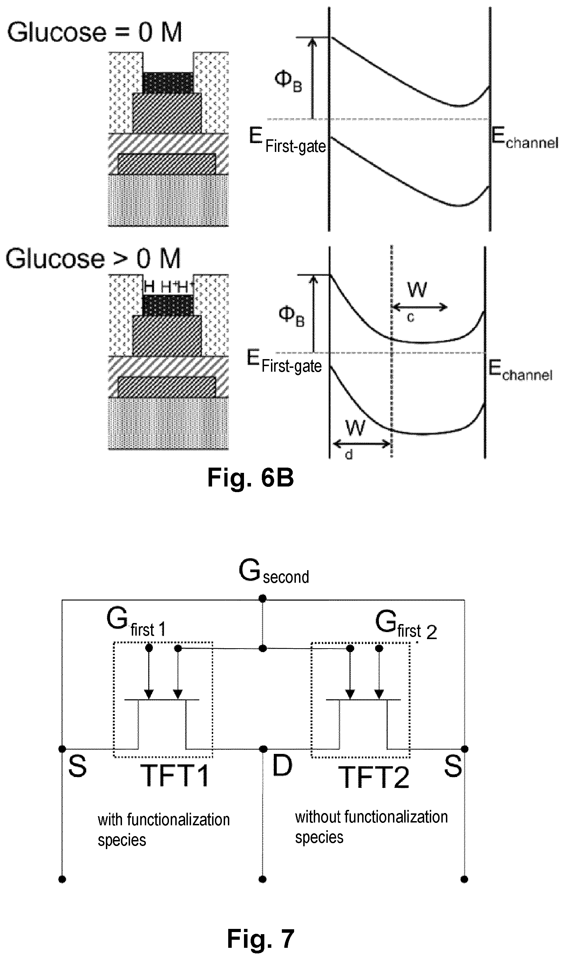

The net result of the above two reactions, according to Equations 1 and 2, is that a stoichiometric quantity of protons is liberated which is directly proportional to the number of glucose molecules oxidized by the enzyme. The protons (since they are charged) affect a depletion region of the detection transistor, and therefore affect the drain-source current ($I_{DS}$) (Fig. 6B). As presented in Fig 6B, at 0% of the target substance (e.g. glucose) in the sample subjected to assay, the channel is nearly pinched off and the current flowing through the channel is low. As the target substance (e.g. glucose) concentration increases, the depletion region thickness (Wd) decreases subsequently increasing the width of the conduction channel (Wc). This leads to lowering the barrier potential and the resistivity between the source electrode and the drain electrode in the channel, which is followed by an increase in the drain-source current ($I_{DS}$).

Figs 2A - 2C depict graphically the electrical measurements which may be taken during the assay with the biosensor comprising the biosensing unit B having the detection transistor 10. As mentioned above, the measurement of the target substance with the detection transistor consists in measuring output characteristics: $I_{DS}$ = f ($V_{DS}$) of the biosensor. The detection transistor (MESFET) with two gate electrodes, the first gate electrode and the second gate electrode, and provided with the channel separating thereof, is under a constant bias of the second gate electrode.

With no polarization of the second gate electrode, at first -gate-source voltage: $V_{GS}$ = 0, and at drain-source voltage: $V_{DS}$=0, the detection transistor is in thermodynamic equilibrium and no current flow occurs. Underneath the first-gate Schottky junction, a depletion layer is present. By increasing $V_{DS}$ > 0 V, the source-drain current ($I_{DS}$) will flow due to the potential difference between the source electrode and the drain electrode, as presented in Fig. 2A. Initially, for low $V_{DS}$, the channel resistance is constant, and $I_{DS}$ depends linearly on $V_{DS}$ according to Ohm's law. With the increase of the $V_{DS}$, the Schottky barrier becomes increasingly reverse biased, and the voltage drops across the channel, and the corresponding potential difference leads to an expansion of the depletion layer at the drain electrode side of the channel. Consequently, the channel narrows at the drain electrode side and leads to the source-drain current ($I_{DS}$) rounding off. At a certain voltage - called saturation drain-source voltage ($V_{DS}$sat.), the depletion layer width equals the channel thickness, i.e. the channel is pinched off, as shown in Fig. 2B. At this drain-source voltage ($V_{DS}$), the source electrode and the drain electrode are completely separated by a reverse-biased depletion region. Despite the pinch-off, and therefore lack of charges, the source-drain current ($I_{DS}$) flows as a consequence of the carriers injection through depletion layer at high electric field in this region. A further increase of $V_{DS}$ shifts the pinch-off point towards the source contact. Increasing the $V_{DS}$ further saturates the diffusive source-drain current ($I_{DS}$sat). An excessive increase of $V_{DS}$ results in the breakdown of the channel. The charge depleted region in the channel can be also controlled by varying the first-gate-source voltage ($V_{GS}$). As $V_{GS}$ becomes more negative the depletion width increases due to the drain - first-gate junction becoming more reversely biased - Fig. 2C. The variation in $V_{GS}$ is in line with changes in the concentration of the monitored target substance such as biochemical species. This in turn causes the channel region to pinch-off at lower drain voltages.

The first gate electrode and the second gate electrode are present in the design of the detection transistor 10 and the reference translator 20, according to the present invention. The second gate electrode 12, 22, 122 as mentioned above is polarized to set the operating point of the device. The first gate electrode of the detection transistor 10 is sensitive to the target substance (when combined with the substrate 31a, 31b). The interaction of the target substance with the functionalization species of the substrate directly affects the channel conductance of the detection transistor - as a function of the monitored target substance. Further, the second gate electrode of the detection transistor is used to control and reduce the feedback capacitance between input and output and thus to set the operating point of the device, thereby, making the measurement and the MESFET itself more stable. The detection transistor 10 with the second gate electrode 12 exhibits higher thermal stability during the detection, thereby providing compensation for the external interfering factors such as temperature.

Further, the biosensor comprising - in addition to the detection transistor 10, the reference transistor 20, provides yet improved compensation of the external factors, such as pH of the sample, which may vary from one user to another. The reference transistor - combined with the detection transistor, can act for example as a pH meter - providing correction of the signal obtained from the detection transistor by the signal obtained from the reference transistor. The depletion region in the channel of the reference transistor is affected by the total charge of the analyte (i.e. the sample), wherein the detection transistor works as described above. Namely, the depletion region in the channel of the detection transistor corresponds to the pH affected by the redox reaction between the functionalization species and the monitored target substance. The difference between the signal of the detection transistor and the signal of the reference transistor provides a deconvolute signal which is highly correlated only with the target substance, for example, glucose. By multiplying the pair of detection and reference transistors, the measurement accuracy can be further increased. To this end, the difference between the value of integrated signals from all the detection transistors and the value of integrated signals from all the reference transistors is calculated. For example, for one pair of detection-reference transistors, one could deconvolute final signal according to Equation 3 :

$$signal = \left(\int(I_{DS_{TFT10}}) - \int(I_{DS_{TFT20}})\right)dI_{DS} \ (3)$$

where:

signal - denotes the signal obtained as the result of the measurement,

$I_{DS_{TFT10}}$ - denotes drain-source current of the detection transistor,

$I_{DS_{TFT20}}$ - denotes drain-source current of the reference transitor.

Further, for more than one pair, e.g. n pairs of detection-reference transistors (wherein each of the pairs comprises one detection transistor and one reference transistor connected to each other as shown in Fig. 3A or 3B), one could deconvolute final signal according to Equation 4:

$$signal = n\left(\int(I_{DS_{TFT10}}) - \int(I_{DS_{TFT20}})\right)dI_{DS} \ (4)$$

Fig. 7 presents the electrical scheme of the two transistors, the detection transistor with the first gate electrode sensitive to the target substance and the reference transistor with the first gate electrode insensitive to the target substance, the transistors are connected by a common second gate electrode 122, which is maintained under constant bias. In other words, the second gate electrodes of both transistors are at the same potential. Similarly, equal voltage is set between the source electrode and the drain electrode of both transistors. The first gate electrodes of both transistors are nonpolarized (floating) and both have direct contact with the target substance during detection. The second gate electrode is connected to the control system (such as a microprocessor) - in the unit C of the biosensor (shown in Fig. 4). Such connection provides stabilization of the operating point and tailoring of the transfer characteristic and stabilizing the operating point.

The difference of the respective signals received from the detection transistor and the reference transistor is next calculated, for example, using a microprocessor with software enabling for ordinary signal processing such as sampling, smoothing etc. Additionally, this data can be wirelessly transferred to the external (mobile) device to process said data. The potential of the common second gate electrode is set according to the the program at the microcontroller unit. The final result of the accomplished assay may be achieved by probing and integrating the drain-source current - from both the detection transistor and the reference transistor, in the time domain and processing (subtracting) in the external application in order to deconvolute value corresponding only to the concentration of the target substance (for example glucose). The resulting value of source-drain current is proportional to the concentration of the target substance being detected.

The biosensor and the biosensing kit according to the present invention provide flexibility for the detection of different biochemicals. The functionalization species contained in the substrate 31a, 31b, can be a glucose oxidase or another biomaterial such as a different enzyme, antibody, DNA, a receptor, or another type of capturing molecule biologically specific for the target substance. When the target substance is in the sample it interacts with the functionalization species - as described above - this, in turn, modulates the conductivity of the channel of the detection transistor and thereby the drain current. The changes that are proportional to the volume of analyzed biochemicals can directly be read by Unit C of the biosensor and can be readily visualized in the display as well as wirelessly transferred to the mobile device, e.g. smartphone, thereby providing fast and reliable assay results. Thus, the assay with the biosensing kit according to the present invention can be carried out directly by the user under domestic conditions, without the aid of professional healthcare workers.

**Example 1 -** a method for preparing the substrate for glucose detection directly on the first gate electrode of the transistor (not in accordance with the present invention).

The glucose oxidase functionalizing solution was prepared by dissolving 15 mg of enzyme powder (glucose oxidase) in 0.15 M phosphate-buffered saline. The obtained glucose oxidase functionalizing solution and a polymeric membrane made of Nafion® ($C_7HF_{13}O_5S \cdot C_2F_4$) were mixed with a volume ratio of 1:1 and stirred for 50 min to form a clear solution. The obtained clear solution was drop cast (however it could be inkjet printed instead) onto the first gate electrode of the MESFET transformer and then dried at 6 °C for 12 h. The obtained transistor had a design according to Fig 1A.

**Example 2** - example of operation.

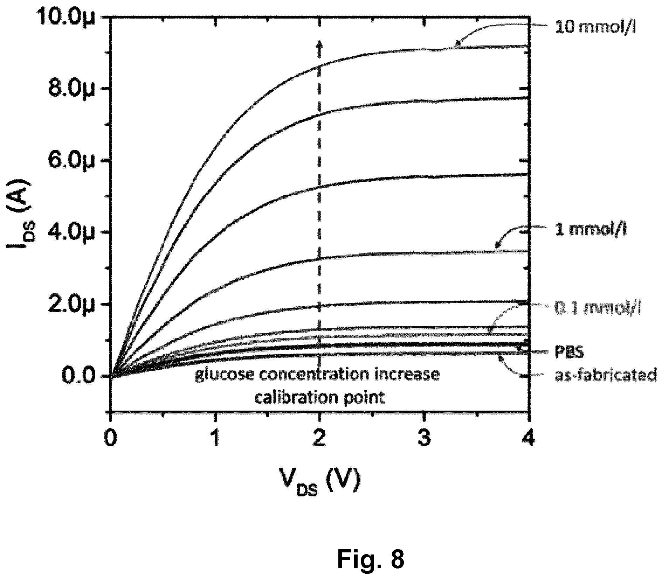

The detection of glucose was carried out by measuring the output characteristics of both detection and reference transistors. Output characteristics of the detection transistor are depicted in Fig. 8.

At 0% of glucose in the analyte the channel is nearly pinched off and the current of 0.6 $\mu$A flows through the channel under $V_{DS} = 2$ V. As the glucose concentration increases, the depletion region thickness decreases subsequently increasing the width of the conduction channel. This leads to the lowering of the resistivity between source and drain in the channel, which is followed by an increase in the drain current reaching 8.0 $\mu$A for glucose concentration equal 10 mmol*l$^{-1}$ under $V_{DS} = 2$ V. Final reading of the biosensor relies on substracting these values from the signal coming from the unfunctionalized reference transistor (which corresponds to plain value of pH).

## Claims

1. A biosensor comprising a detection transistor (10) of a metal-semiconductor field-effect (MESFET) type, for detecting of a target substance, wherein the detection transistor (10) comprises:

   - an isolating substrate (16),
   - a source electrode (13),
   - a drain electrode (14),
   - a first gate electrode (11), and
   - a channel (15) for transmitting a drain-source current ($I_{DS}$) between the source electrode (13) and the drain electrode (14),
   - the channel (15) is made of an oxide semiconductor;
   - the source electrode (13) and the drain electrode (14) are arranged on the channel (15) that is arranged on the isolating substrate (16);
   - and the detection transistor (10) further comprises:

     - a sensitive substrate ( 31b) connected to the first gate electrode (11) and comprising functionalization species for bonding the target substance, such that the first gate electrode (11) connected with said substrate ( 31b) is sensitive to the target substance,

   wherein the detection transistor (10) further comprises:

     - a conductive path,
     - a disposable strip,

     - a second gate electrode (12) arranged on the isolating substrate (16), physically separated from the first gate electrode (11) by the channel (15), positioned oppositely to the first gate electrode (11) and configured to modulate the drain-source current (IDS) of the channel (15),

   wherein the sensitive substrate (31b) is detachably connected to the first gate electrode (11) via the conductive path, and

wherein the sensitive substrate (31b) is arranged on the disposable strip comprising a sheet of material with the functionalization species.

2. The biosensor according to claim 1, wherein the second gate electrode (12) is positioned along the channel (15) in an area to which at the opposite side of the channel there is adjacent the first gate electrode (11) and optionally only one of the source electrode (13) or the drain electrode (14).

3. The biosensor according to any of the preceding claims further comprising an encapsulation (17) arranged such that it exposes the first gate electrode (11).

4. The biosensor according to any of the claims from 1 to 3 wherein the conductive path is selected from the group consisting of conductive film, wiring, junction connector, and electrolytic connection.

5. The biosensor according to any of the preceding claims, wherein the isolating substrate (16) is a plastic foil.

6. The biosensor according to any of the preceding claims further comprising a reference transistor (20) of a metal-semiconductor field-effect (MESFET) type, the reference transistor (20) comprising:

   - a source electrode (23),
   - a drain electrode (24),
   - a first gate electrode (21) connected with an insensitive substrate (32b) without the functionalization species of the sensitive substrate (31b), such that the first gate electrode (21) connected with said insensitive substrate (32b) is insensitive to the target substance,
   - a channel (25) made of an oxide semiconductor for transmitting a drain-source current ($I_{DS}$) between the source electrode (23) and the drain electrode (24), and
   - a second gate electrode (22) physically separated from the first gate electrode (21) by the channel (25),
   - wherein the detection transistor (10) and the reference transistor (20) are both placed on the isolating substrate (16) and are electrically connected to one another by the second gate electrodes (12, 22).

7. The biosensor according to claim 6 wherein the second gate electrode (12) of the detection transistor (10) and the second gate electrode (22) of the reference transistor (20) are arranged as a common second gate electrode (122) of both transistors (10, 20), formed from a coherent piece of material.

8. The biosensor according to claim 6 or 7 wherein the detection transistor (10) and the reference transistor (20) are covered with a common encapsulation (127).

9. The biosensor according to claim 7 or 8 wherein the common second gate electrode (122) extends from the channel (15) of the detection transistor (10) to the channel (25) of the reference transistor (20), wherein said channels (15, 25) separate the common second gate electrode (122) from the first gate electrodes (11, 21) of both transistors (10, 20).

10. A biosensing device comprising the biosensor according to any of previous claims and further comprising:

    - a control unit (C) for processing measured data;
    - a power supply unit (D) for powering the device; and
    - a data display unit (E) for visualizing a detection result.

11. A biosensing kit comprising:

    - the biosensor according to any of claims from 1 to 5, and
    - a disposable substrate unit (A) comprising:

        - the sensitive substrate (31b) with functionalization species sensitive to the target substance, detachably connectable with the first gate electrode (11) of the detection transistor (10).

12. A biosensing kit comprising:

    - the biosensor according to any of the claims from 6 to 9, and:

- a disposable substrate unit (A) comprising:

- the sensitive substrate (31b) with functionalization species sensitive to the target substance, detachably connectable with the first gate electrode (11) of the detection transistor (10), and
- the insensitive substrate (32b) without functionalization species sensitive to the target substance, detachably connectable with the first gate electrode (21) of the reference transistor (20).

**Patentansprüche**

1. Biosensor, der einen Detektionstransistor (10) vom Typ eines Metall-Halbleiter-Feldeffekttransistors (MESFET) zum Nachweis einer Zielsubstanz umfasst, wobei der Detektionstransistor (10) umfasst:

- ein isolierendes Substrat (16),
- eine Source-Elektrode (13),
- eine Drain-Elektrode (14),
- eine erste Gate-Elektrode (11) und
- einen Kanal (15) zum Leiten eines Drain-Source-Stroms (IDS) zwischen der Source-Elektrode (13) und der Drain-Elektrode (14),
- wobei der Kanal (15) aus einem Oxidhalbleiter besteht;
- wobei die Source-Elektrode (13) und die Drain-Elektrode (14) auf dem Kanal (15) angeordnet sind, der auf dem isolierenden Substrat (16) angeordnet ist;
- und wobei der Detektionstransistor (10) ferner umfasst:

- ein empfindliches Substrat (31b), das mit der ersten Gate-Elektrode (11) verbunden ist und Funktionalisierungsspezies zum Binden der Zielsubstanz umfasst, so dass die mit dem Substrat (31b) verbundene erste Gate-Elektrode (11) gegenüber der Zielsubstanz empfindlich ist,

wobei der Detektionstransistor (10) ferner umfasst:

- einen leitfähigen Pfad,
- einen Einwegstreifen,
- eine zweite Gate-Elektrode (12), die auf dem isolierenden Substrat (16) angeordnet ist, physisch durch den Kanal (15) von der ersten Gate-Elektrode (11) getrennt ist, der ersten Gate-Elektrode (11) gegenüberliegt und so konfiguriert ist, dass sie den Drain-Source-Strom (IDS) des Kanals (15) moduliert,

wobei das empfindliche Substrat (31b) über den leitfähigen Pfad lösbar mit der ersten Gate-Elektrode (11) verbunden ist, und
wobei das empfindliche Substrat (31b) auf dem Einwegstreifen angeordnet ist, der eine Materialbahn mit den Funktionalisierungsspezies umfasst.

2. Der Biosensor nach Anspruch 1, wobei die zweite Gate-Elektrode (12) entlang des Kanals (15) in einem Bereich positioniert ist, an dessen gegenüberliegender Seite die erste Gate-Elektrode (11) und optional nur eine der Source-Elektrode (13) oder der Drain-Elektrode (14) angrenzt.

3. Der Biosensor nach einem der vorstehenden Ansprüche, der ferner eine Verkapselung (17) umfasst, die so angeordnet ist, dass sie die erste Gate-Elektrode (11) freilegt.

4. Der Biosensor nach einem der Ansprüche 1 bis 3, wobei der leitfähige Pfad aus der Gruppe ausgewählt ist, die aus einer leitfähigen Schicht, einer Verdrahtung, einem Verbindungsstecker und einer elektrolytischen Verbindung besteht.

5. Der Biosensor nach einem der vorstehenden Ansprüche, wobei das isolierende Substrat (16) eine Kunststofffolie ist.

6. Der Biosensor nach einem der vorstehenden Ansprüche, der ferner einen Referenztransistor (20) vom Typ eines Metall-Halbleiter-Feldeffekttransistors (MESFET) umfasst, wobei der Referenztransistor (20) umfasst:

- eine Source-Elektrode (23),

- eine Drain-Elektrode (24),
- eine erste Gate-Elektrode (21), die mit einem unempfindlichen Substrat (32b) verbunden ist, das keine Funktionalisierungsspezies des empfindlichen Substrats (31b) aufweist, so dass die mit dem unempfindlichen Substrat (32b) verbundene erste Gate-Elektrode (21) gegenüber der Zielsubstanz unempfindlich ist,
- einen Kanal (25) aus einem Oxidhalbleiter zum Leiten eines Drain-Source-Stroms (IDS) zwischen der Source-Elektrode (23) und der Drain-Elektrode (24), und
- eine zweite Gate-Elektrode (22), die durch den Kanal (25) physisch von der ersten Gate-Elektrode (21) getrennt ist,
- wobei der Detektionstransistor (10) und der Referenztransistor (20) beide auf dem isolierenden Substrat (16) angeordnet sind und über die zweiten Gate-Elektroden (12, 22) elektrisch miteinander verbunden sind.

7. Der Biosensor nach Anspruch 6, wobei die zweite Gate-Elektrode (12) des Detektionstransistors (10) und die zweite Gate-Elektrode (22) des Referenztransistors (20) als gemeinsame zweite Gate-Elektrode (122) beider Transistoren (10, 20) angeordnet sind, die aus einem zusammenhängenden Materialstück gebildet ist.

8. Der Biosensor nach Anspruch 6 oder 7, wobei der Detektionstransistor (10) und der Referenztransistor (20) mit einer gemeinsamen Verkapselung (127) abgedeckt sind.

9. Der Biosensor nach Anspruch 7 oder 8, wobei sich die gemeinsame zweite Gate-Elektrode (122) vom Kanal (15) des Detektionstransistors (10) zum Kanal (25) des Referenztransistors (20) erstreckt, wobei die genannten Kanäle (15, 25) die gemeinsame zweite Gate-Elektrode (122) von den ersten Gate-Elektroden (11, 21) beider Transistoren (10, 20) trennen.

10. Biosensoreinrichtung, umfassend den Biosensor gemäß einem der vorstehenden Ansprüche und ferner umfassend:

- eine Steuereinheit (C) zur Verarbeitung gemessener Daten;
- eine Stromversorgungseinheit (D) zur Versorgung der Einrichtung; und
- eine Datenanzeigeeinheit (E) zur Visualisierung eines Detektionsergebnisses.

11. Biosensor-Kit, umfassend:

- den Biosensor gemäß einem der Ansprüche 1 bis 5, und
- eine Einweg-Substrateinheit (A), umfassend:

- das empfindliche Substrat (31b) mit Funktionalisierungsspezies, die gegenüber der Zielsubstanz empfindlich sind, das lösbar mit der ersten Gate-Elektrode (11) des Detektionstransistors (10) verbindbar ist.

12. Biosensor-Kit, umfassend:

- den Biosensor gemäß einem der Ansprüche 6 bis 9, und
- eine Einweg-Substrateinheit (A), umfassend:

- das empfindliche Substrat (31b) mit Funktionalisierungsspezies, die gegenüber der Zielsubstanz empfindlich sind, das lösbar mit der ersten Gate-Elektrode (11) des Detektionstransistors (10) verbindbar ist, und
- das unempfindliche Substrat (32b) ohne Funktionalisierungsspezies, die gegenüber der Zielsubstanz empfindlich sind, das lösbar mit der ersten Gate-Elektrode (21) des Referenztransistors (20) verbindbar ist.

**Revendications**

1. Biocapteur comprenant un transistor de détection (10) de type à effet de champ métal-semi-conducteur (MESFET), destiné à la détection d'une substance cible, dans lequel le transistor de détection (10) comprend :

- un substrat isolant (16),
- une électrode de source (13),
- une électrode de drain (14),
- une première électrode de grille (11), et
- un canal (15) destiné à transmettre un courant drain-source (IDS) entre l'électrode de source (13) et l'électrode de drain (14),
- le canal (15) est constitué d'un semi-conducteur à oxyde ;
- l'électrode de source (13) et l'électrode de drain (14) sont disposées sur le canal (15) qui est disposé sur le substrat isolant (16) ;
- et le transistor de détection (10) comprend en outre :

- un substrat sensible (31b) relié à la première électrode de grille (11) et comprenant des espèces de fonctionnalisation permettant la liaison de la substance cible, de telle sorte que la première électrode de grille (11) reliée audit substrat (31b) soit sensible à la substance cible,

dans lequel le transistor de détection (10) comprend en outre :

- un chemin conducteur,
- une bande jetable,
- une deuxième électrode de grille (12) disposée sur le substrat isolant (16), physiquement séparée de la première électrode de grille (11) par le canal (15), positionnée à l'opposé de la première électrode de grille (11) et configurée pour moduler le courant drain-source (IDS) du canal (15),

dans lequel le substrat sensible (31b) est relié de manière amovible à la première électrode de grille (11) via le chemin conducteur, et
dans lequel le substrat sensible (31b) est disposé sur la bande jetable comprenant une feuille de matériau comportant les espèces de fonctionnalisation.

2. Le biocapteur selon la revendication 1, dans lequel la deuxième électrode de grille (12) est positionnée le long du canal (15) dans une zone où, du côté opposé du canal, se trouve à proximité la première électrode de grille (11) et, éventuellement, uniquement l'une de l'électrode de source (13) ou de l'électrode de drain (14).

3. Le biocapteur selon l'une quelconque des revendications précédentes, comprenant en outre un encapsulage (17) agencé de telle sorte qu'il expose la première électrode de grille (11).

4. Le biocapteur selon l'une quelconque des revendications 1 à 3, dans lequel le chemin conducteur est choisi dans le groupe constitué d'un film conducteur, d'un câblage, d'un connecteur de jonction et d'une connexion électrolytique.

5. Le biocapteur selon l'une quelconque des revendications précédentes, dans lequel le substrat isolant (16) est une feuille plastique.

6. Le biocapteur selon l'une quelconque des revendications précédentes, comprenant en outre un transistor de référence (20) de type à effet de champ métal-semi-conducteur (MESFET), le transistor de référence (20) comprenant :

- une électrode de source (23),
- une électrode de drain (24),
- une première électrode de grille (21) reliée à un substrat insensible (32b) dépourvu des espèces de fonctionnalisation du substrat sensible (31b), de telle sorte que la première électrode de grille (21) reliée audit substrat insensible (32b) soit insensible à la substance cible,
- un canal (25) constitué d'un semi-conducteur à oxyde pour transmettre un courant drain-source (IDS) entre l'électrode de source (23) et l'électrode de drain (24), et
- une deuxième électrode de grille (22) physiquement séparée de la première électrode de grille (21) par le canal (25),
- dans lequel le transistor de détection (10) et le transistor de référence (20) sont tous deux placés sur le substrat isolant (16) et sont reliés électriquement l'un à l'autre par les deuxièmes électrodes de grille (12, 22).

7. Le biocapteur selon la revendication 6, dans lequel la deuxième électrode de grille (12) du transistor de détection (10) et la deuxième électrode de grille (22) du transistor de référence (20) sont agencées sous la forme d'une deuxième électrode de grille commune (122) aux deux transistors (10, 20), formée à partir d'une pièce de matériau d'un seul tenant.

8. Le biocapteur selon la revendication 6 ou 7, dans lequel le transistor de détection (10) et le transistor de référence (20) sont recouverts d'un encapsulage commun (127).

9. Le biocapteur selon la revendication 7 ou 8, dans lequel la deuxième électrode de grille commune (122) s'étend du canal (15) du transistor de détection (10) au canal (25) du transistor de référence (20), lesdits canaux (15, 25) séparant la deuxième électrode de grille commune (122) des premières électrodes de grille (11, 21) des deux transistors (10, 20).

10. Dispositif de biodétection comprenant le biocapteur selon l'une quelconque des revendications précédentes et comprenant en outre :

    - une unité de commande (C) pour le traitement des données mesurées ;
    - une unité d'alimentation (D) pour l'alimentation du dispositif ; et
    - une unité d'affichage de données (E) pour la visualisation d'un résultat de détection.

11. Kit de biodétection comprenant :

    - le biocapteur selon l'une quelconque des revendications 1 à 5, et
    - une unité de substrat jetable (A) comprenant :

        - le substrat sensible (31b) comportant des espèces de fonctionnalisation sensibles à la substance cible, pouvant être relié de manière amovible à la première électrode de grille (11) du transistor de détection (10).

12. Kit de biodétection comprenant :

    - le biocapteur selon l'une quelconque des revendications 6 à 9, et :
    - une unité de substrat jetable (A) comprenant :

        - le substrat sensible (31b) comportant des espèces de fonctionnalisation sensibles à la substance cible, pouvant être relié de manière amovible à la première électrode de grille (11) du transistor de détection (10), et
        - le substrat insensible (32b) dépourvu d'espèces de fonctionnalisation sensibles à la substance cible, pouvant être relié de manière amovible à la première électrode de grille (21) du transistor de référence (20).

Fig. 1A

Fig. 1B

VG=0    VDS<VDSsat.

Fig. 2A

VG=0    VDS=VDSsat.

## Fig. 2B

VG<VTH    VDS.

## Fig. 2C

10    20

31a    11    32a    21    27

24

25

26

12    23    22

## Fig. 3A

10    20

127

24

25

126

122    23    21

**Fig. 3B**

10    20

Unit A    Unit B

Unit C    Unit D

Unit E

32b    31b

**Fig. 4**

Phase I

Unit A

Phase III

Phase II

Phase IV

GLUCOSE
119 mg/dL

Fig. 5

Conductive Path

Unit A

Unit B

Unit C

Unit D

Unit E

Fig. 6A

Glucose = 0 M

$\Phi_B$

$E_{First\text{-}gate}$

$E_{channel}$

Glucose > 0 M

H H$^+$H$^+$

$\Phi_B$

$E_{First\text{-}gate}$

$E_{channel}$

$W_c$

$W_d$

## Fig. 6B

$G_{second}$

$G_{first\ 1}$                                    $G_{first\ 2}$

S                          D                          S

TFT1                                    TFT2

with functionalization
species

without functionalization
species

## Fig. 7

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110132773 A1
- US 2010194409 A1
- US 2016202208 A1
- US 2015276667 A1

**Non-patent literature cited in the description**

- **OH JESEUNG et al.** *Biosensors and Bioelectronics*, 2013, vol. 50, 345-350
- **JAKUB KACZMARSKI et al.** IGZO MESFET with enzyme-modified Schottky gate electrode for glucose sensing. *Japanese Journal of Applied Physics*, 2019, vol. 58 (9), 090603